# EUROPEAN PATENT APPLICATION

(11) **EP 2 942 015 A2**
(43) Date of publication of application: **11.11.2015**
(21) Application number: 14199662.9
(22) Date of filing: 22.12.2014
(51) Int. Cl.: A61B 17/00

(54) **Manual retraction tool for use with an electromechanical surgical device**

(30) Priority: 17.04.2014 US 201461980743 P; 03.12.2014 US 201414559308
(71) Applicant: Covidien LP, Mansfield, MA 02048 (US)
(72) Inventor: Zergiebel, Earl M., Guilford, Connecticut 06430 (US); Chowaniec, David, Rocky Hill, Connecticut 06067 (US); Williams, Ryan V., New Hartford, Connecticut 06057 (US); Subramanian, Anand, Stamford, Connecticut 06902 (US)
(74) Representative: Soames, Candida Jane

(57) **Abstract**

A manual retraction tool for use with an electromechanical surgical assembly includes a handle and a shaft. The handle includes an elongated handle body having a first end and a second end, and a channel recessed within at least a portion of the handle body. The shaft includes an elongated shaft body having a tip dimensioned to be inserted into a recess of a connector sleeve of an adapter of the electromechanical surgical assembly and to effect rotation of the connector sleeve upon rotation of the shaft, and a second end pivotally connected to the handle body. The shaft body is movable between a first position wherein the shaft is at least partially disposed within the channel of the handle body and longitudinally aligned with the handle body, and a second position wherein the shaft body is perpendicular to the handle body.

## Description

### BACKGROUND

This application claims the benefit of and priority to U.S. Provisional Patent Application No. 61/980,743, filed April 17, 2014, the entire disclosure of which is incorporated by reference herein.

### BACKGROUND

### 1. Technical Field

The present disclosure relates to surgical apparatuses, devices and/or systems for performing endoscopic surgical procedures. More specifically, the present disclosure relates to manual retraction tools configured for use with electromechanical, hand-held surgical apparatuses, to kits, and methods of use thereof.

### 2. Background of Related Art

A number of surgical device manufacturers have developed product lines with proprietary drive systems for operating and/or manipulating electromechanical surgical devices. In many instances the electromechanical surgical devices include a handle assembly, which is reusable, and disposable loading units and/or single use loading units or the like that are selectively connected to the handle assembly prior to use and then disconnected from the handle assembly following use in order to be disposed of or, in some instances, sterilized for re-use.

In certain instances, an adapter is used to interconnect an electromechanical surgical device with an end effector, e.g., any one of a number of surgical loading units, to establish a mechanical and/or electrical connection therebetween. The adapter, or different adapters, may be attached and detached to an electromechanical surgical device during a surgical procedure.

These electromechanical surgical devices offer some advantages over purely mechanical devices.

### SUMMARY

The present disclosure is directed to a manual retraction tool that can be used in lieu of a surgical device of an electromechanical surgical system to provide a clinician with a means to manually operate and perform standard operations of an end effector associated with the surgical device, for example, to complete or reverse actuation, articulation, and/or rotation functions of the end effector.

In one aspect of the present disclosure, a manual retraction tool is configured for use with an electromechanical surgical assembly including: a surgical device including at least one drive connector disposed within a connecting portion, an adapter including at least one connector sleeve defining a recess therein configured to receive the drive connector of the surgical device; and an end effector attached to the adapter. The manual retraction tool includes a handle including an elongated handle body having a first end and a second end, and a channel recessed within at least a portion of the handle body. The manual retraction tool also includes a shaft including an elongated shaft body having a tip dimensioned to be inserted into the recess of a connector sleeve of an adapter and to effect rotation of a connector sleeve upon rotation of the shaft, and a second end pivotally connected to the handle body. The shaft body is movable between a first position wherein the shaft is at least partially disposed within the channel of the handle body and longitudinally aligned with the handle body, and a second position wherein the shaft body is perpendicular to the handle body.

In some embodiments, the second end of the shaft body is pivotally connected to a central portion of the handle body. In certain embodiments, the channel extends along an entire length of the handle body, and the shaft body is movable to a third position that mirrors the first position. In some other embodiments, the second end of the shaft is pivotally connected the second end of the handle body. In certain embodiments, the shaft is movable to a third position in which a majority of the shaft extends out of the second end of the shaft body.

The handle body may include through-holes transversely spaced and longitudinally aligned on opposing sides of the channel. The second end of the shaft body may include a through-hole that is aligned with the through-holes of the handle. A connector member may extend transversely through the through-hole of the shaft body and the through-holes of the handle body to pivotally connect the shaft to the handle. In some embodiments, the through-holes of the handle body are disposed within a central portion of the handle body. In some other embodiments, the through-holes of the handle body are disposed within the second end of the handle body.

In some embodiments, the tip of the shaft may extend out of the first end of the handle body when in the first position. In certain embodiments, the tip of the shaft extends a distance that is a same distance that the drive connector extends from the connecting portion of the surgical device. In some other embodiments, the tip of the shaft is retained within the channel of the handle body when in the first position.

The first end of the handle body may include retaining features for retaining the shaft in the first position. The handle body may include retaining members for retaining the shaft in the second position.

In embodiments, the manual retraction tool may further include a handle assembly including the handle described above as a first handle, and a second handle. The first and second handles include an elongated first handle body and an elongated second handle body, respectively, each of the first and second handle bodies having a first end and a second end, and a channel recessed within a surface thereof. The second handle is pivotally connected to the first handle such that the shaft, the first handle, and the second handle are all independently pivotable with respect to each other.

The handle assembly may be pivotable to a closed position in which the first and second handles mate with each other and longitudinal axes of the first and second handle bodies are coincident with each other. In embodiments, the first and second handle bodies include complementary engagement parts for releasably retaining the first and second handles in the closed position. The handle assembly may be pivotable to a semi-open position in which the first and second handle bodies are orthogonal to each other. The handle assembly may also be pivotable to a fully open position in which the first and second handle bodies longitudinally extend along an axis.

In a method of using the manual retraction tool described above, the tip of the shaft is inserted into a connector sleeve of an adapter, and the manual retraction tool is rotated to effect rotation of a connector sleeve. The surgical device may need to be disconnected from the adapter prior to inserting the tip of the shaft into the connector sleeve. The manual retraction tool may be rotated by gripping the handle. In embodiments in which the tip of the shaft extends out of the first end of the handle body, the tip may extend a distance that the same distance a drive connector extends from the connecting portion of the surgical device, such that when inserting the tip of the shaft into the connector sleeve the first end of the handle body may be placed flush against the adapter. The manual retraction tool may be utilized in the first or second positions.

In another aspect of the present disclosure, a manual retraction tool includes a shaft, a handle assembly, and a connector member. The shaft includes an elongated shaft body having a tip dimensioned to be received within a recess of a connector sleeve of an adapter and configured to rotate the connector sleeve. The handle assembly includes a movable first handle and a fixed second handle. The first handle includes an elongated first handle body having a first end and a second end. The first handle body includes a channel recessed within at least a portion of a surface thereof. The shaft body is disposed within the channel and the tip of the shaft body longitudinal extends out of the first end of the first handle. The second handle includes an elongated second handle body having a first end including a notch at an outermost end thereof and a second end. The connector member includes a pair of elongated rods pivotally attached to the first handle and securely attached to the second handle. The first handle is movable from a retracted position in which the tip of the shaft is disposed within the notch of the second handle and an extended position in which the first handle is pivoted 180° relative to the second handle. The first handle may be movable to a semi-expanded position in which the first and second handles are orthogonal to each other and longitudinally spaced.

A kit may contain a manual retraction tool including a handle pivotally connected to a shaft, and an adapter including an outer knob housing and an outer tube extending distally from the outer knob housing. The handle of the manual retraction tool includes an elongated handle body and the shaft of the manual retraction tool includes an elongated shaft body having a tip. The shaft body is movable between a first position wherein the shaft body is longitudinally aligned with the handle body, and a second position wherein the shaft body is perpendicular to the handle body. The kit may further include packaging containing the manual retraction tool and the adapter therein. In embodiments, the packaging includes a tray. In such embodiments, the outer diameter of the handle of the manual retraction tool may have the same diameter as the outer diameter of the outer tube of the adapter for tray compatibility.

### BRIEF DESCRIPTION OF THE DRAWINGS

Embodiments of the present disclosure are described herein with reference to the accompanying drawings, wherein:
FIG. 1 is a perspective view of an electromechanical surgical system in accordance with an embodiment of the present disclosure including an adapter interconnected between an electromechanical surgical device and an end effector;
FIG. 2 is a perspective view illustrating an attachment of a proximal end of the adapter to a distal end of the electromechanical surgical device of FIG. 1;
FIG. 3 is a perspective view of a manual retraction tool in accordance with an embodiment of the present disclosure illustrating a shaft in a first position;
FIG. 4 is a perspective view of the shaft of FIG. 3;
FIG. 5 is an enlarged view of the indicated area of detail of FIG. 3;
FIG. 6 is an enlarged top view of the indicated area of detail of FIG. 3;
FIG. 7 is a perspective view of the manual retraction tool of FIG. 3 illustrating the shaft in a second position;
FIG. 8 is a top view of a manual retraction tool in accordance with an alternative embodiment of the present disclosure illustrating a shaft in a first position;
FIG. 9 is a perspective view of the manual retraction tool of FIG. 8 illustrating the shaft in a second position;
FIG. 10 a perspective view of a manual retraction tool in accordance with another alternative embodiment of the present disclosure illustrating a shaft in a first position;
FIG. 11 is a perspective view of the manual retraction tool of FIG. 10 illustrating the shaft in a second position;
FIG. 12 is a perspective view of a manual retraction tool in accordance with another embodiment of the present disclosure illustrating a shaft in a first position;
FIG. 13 is a perspective view of the manual retraction tool of FIG. 12 illustrating the shaft in a second position;
FIG. 14 is a perspective view of the manual retraction tool of FIG. 12 illustrating the shaft in a third position;
FIG. 15 is a top view of a manual retraction tool in accordance with an alternative embodiment of the present disclosure illustrating a shaft in a first position;
FIG. 16 is a perspective view of the manual retraction tool of FIG. 15 illustrating the shaft in a second position;
FIG. 17 is a perspective view of the manual retraction tool of FIG. 15 illustrating the shaft in a third position;
FIG. 18 is a perspective view of a manual retraction tool in accordance with another embodiment of the present disclosure illustrating a shaft in a first position and a handle assembly in a closed position;
FIG. 19 is a perspective view of the manual retraction tool of FIG. 18 illustrating the shaft in the first position and the handle assembly in a semi-open position;
FIG. 20 is a perspective view of the manual retraction tool of FIG. 18 illustrating the shaft in a first position and the handle assembly in a fully open position;
FIG. 21 is a perspective view of the manual retraction tool of FIG. 20 illustrating a first handle of the handle assembly in wire frame;
FIG. 22 is a perspective view of the manual retraction tool of FIG. 18 illustrating the shaft in a second position and the handle assembly in a fully open position;
FIG. 23 is a top view of a manual retraction tool in accordance with yet another embodiment of the present disclosure illustrating a shaft in a retracted position;
FIG. 24 is a side view of the manual retraction tool of FIG. 23 illustrating the shaft in a semi-extended position;
FIG. 25 is a top view of the manual retraction tool of FIG. 23 illustrating the shaft in a fully extended position; and
FIG. 26 is a schematic illustration of a kit according to the present disclosure.

### DETAILED DESCRIPTION OF EMBODIMENTS

Embodiments of the presently disclosed manual retraction tools for use with electromechanical surgical systems are described in detail with reference to the drawings, in which like reference numerals designate identical or corresponding elements in each of the several views. As used herein, the term "distal" refers to a portion of a structure that is farther from a clinician, while the term "proximal" refers to a portion of a structure that is closer to a clinician. As used herein, the term "clinician" refers to a doctor, nurse, or other care provider and may include support personnel.

The exemplary embodiments of a manual retraction tool, kits, and methods of use, are disclosed and discussed in terms of a tool for actuating an adapter of an electromechanical surgical system to effect operation of an end effector. However, it should be appreciated that the present disclosure may be used in a range of electrosurgical devices.

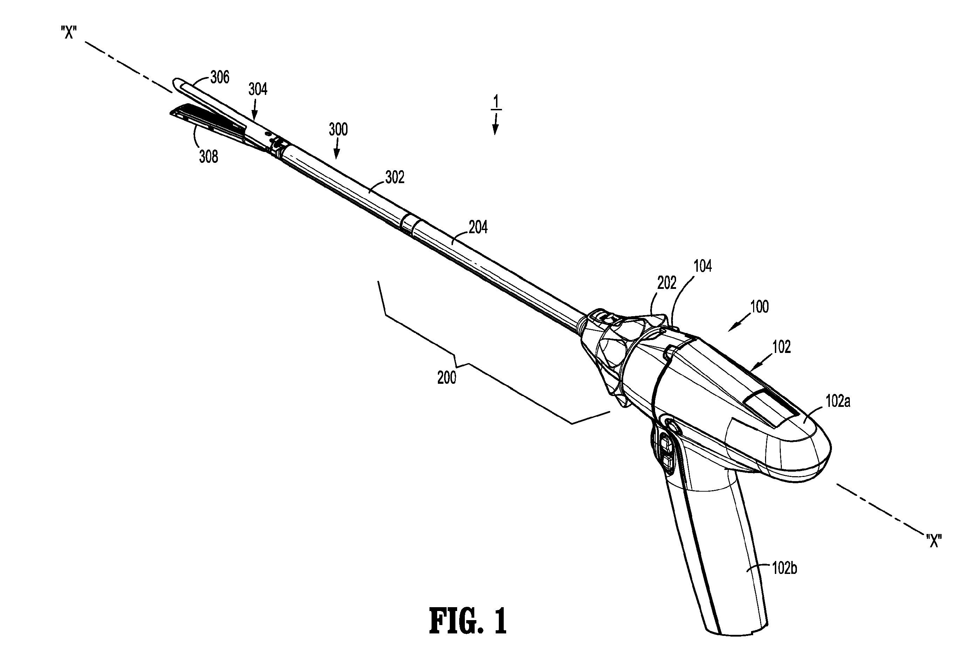

As illustrated in FIG. 1, an exemplary electromechanical surgical system 1 for use with a manual retraction tool of the present disclosure includes a surgical device 100 in the form of a powered handheld electromechanical instrument or device that is configured for selective connection with an adapter 200, and, in turn, the adapter 200 is configured for selective connection with an end effector 300 (e.g., multiple- or single-use loading units, etc.). A plurality of different end effectors may be connected to adapter 200, each end effector being configuration for actuation and manipulation by the powered handheld electromechanical surgical device 100. For the purposes of discussion, the end effectors will be discussed in terms of surgical loading units; however, electromechanical surgical system 1 can be used with a variety of end effectors 300 within the purview of those skilled in the art, such as, for example, clamping jaws and cutting tools.

The surgical device 100 includes a handle housing 102 including a circuit board (not shown) and a drive mechanism (not shown) situated therein. The circuit board is configured to control the various operations of surgical device 100. Handle housing 102 defines a cavity therein (not shown) for selective removable receipt of a battery (not shown). The battery is configured to supply power to any of the electrical components of surgical device 100.

Handle housing 102 includes an upper housing portion 102a which houses various components of surgical device 100, and a lower hand grip portion 102b extending from upper housing portion 102a. Lower hand grip portion 102b may be disposed distally of a proximal-most end of upper housing portion 102a. The location of lower housing portion 102b relative to upper housing portion 102a is selected to balance a weight of surgical device 100 that is connected to or supporting adapter 200 and/or loading unit 300.

Handle housing 102 provides a housing in which the drive mechanism is situated and supports a plurality of finger-actuated control buttons, rocker devices, and the like for activating various functions of surgical device 100. The drive mechanism is configured to drive shafts and/or gear components in order to perform the various operations of surgical device 100. In particular, the drive mechanism is configured to drive shafts and/or gear components in order to selectively move a tool assembly 304 of loading unit 300 relative to a proximal body portion 302 of loading unit 300, to rotate loading unit 300 about a longitudinal axis "X" relative to handle housing 102, and to move/approximate an anvil assembly 306 and a cartridge assembly 308 of loading unit 300 relative to one another, and/or to fire a stapling and cutting cartridge within cartridge assembly 308 of loading unit 300.

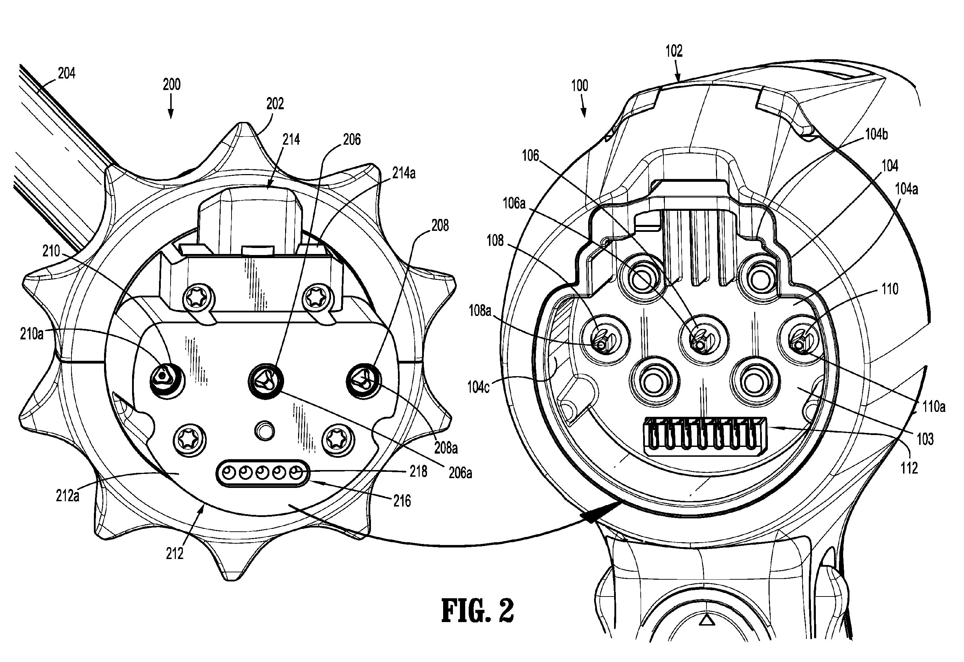

As shown in FIG. 2, in conjunction with FIG. 1, handle housing 102 defines a connecting portion 104 configured to accept a corresponding drive coupling assembly 212 of adapter 200. Specifically, connecting portion 104 of surgical device 100 has a distal facing recess 104a that receives a proximal facing cap 212a of drive coupling assembly 212 of adapter 200 when adapter 200 is mated to surgical device 100. Connecting portion 104 houses three rotatable drive connectors 106, 108, 110 which are arranged in a common plane or line with one another.

When adapter 200 is mated to surgical device 100, each of rotatable drive connectors 106, 108, 110 of surgical device 100 couples with a corresponding rotatable connector sleeve 206, 208, 210 of adapter 200. In this regard, the interface between corresponding first drive connector 106 and first connector sleeve 206, the interface between corresponding second drive connector 108 and second connector sleeve 208, and the interface between corresponding third drive connector 110 and third connector sleeve 210 are keyed such that rotation of each of drive connectors 106, 108, 110 of surgical device 100 causes a corresponding rotation of corresponding connector sleeve 206, 208, 210 of adapter 200. Each of drive connectors 106, 108, 110 includes a tri-lobe tip 106a, 108a, 110a extending distally beyond a wall 103 of connecting portion 104, and each of connector sleeve 206, 208, 210 includes a tri-lobe recess 206a, 208a, 210a for receiving corresponding tip-lobe tip 106a, 108a, 110a. However, it should be understood that the shape of the tips and the recesses may be any shape that are complementary to each other and/or allow the drive connectors to rotate the connector sleeves.

The mating of drive connectors 106, 108, 110 of surgical device 100 with connector sleeves 206, 208, 210 of adapter 200 allows rotational forces to be independently transmitted via each of the three respective connector interfaces. Drive connectors 106, 108, 110 of surgical device 100 are configured to be independently rotated by the drive mechanism of surgical device 100. In this regard, a function selection module (not shown) of the drive mechanism selects which drive connector or connectors 106, 108, 110 of surgical device 100 is to be driven by the motor of surgical device 100.

Since each of drive connectors 106, 108, 110 of surgical device 100 has a keyed and/or substantially non-rotatable interface with respective connector sleeves 206, 208, 210 of adapter 200, when adapter 200 is coupled to surgical device 100, rotational force(s) are selectively transferred from the drive mechanism of surgical device 100 to adapter 200.

The selective rotation of drive connector(s) 106, 108, 110 of surgical device 100 allows surgical device 100 to selectively actuate different functions of loading unit 300. For example, selective and independent rotation of first drive connector 106 of surgical device 100 corresponds to the selective and independent opening and closing of tool assembly 304 of loading unit 300, and driving of a stapling/cutting component of tool assembly 304 of loading unit 300. As an additional example, the selective and independent rotation of second drive connector 108 of surgical device 100 corresponds to the selective and independent articulation of tool assembly 304 of loading unit 300 transverse to longitudinal axis "X". Additionally, for instance, the selective and independent rotation of third drive connector 110 of surgical device 100 corresponds to the selective and independent rotation of loading unit 300 about longitudinal axis "X" relative to handle housing 102 of surgical device 100.

Adapter 200 includes an outer knob housing 202 and an outer tube 204 extending from a distal end of knob housing 202. Knob housing 202 and outer tube 204 are configured and dimensioned to house the components of adapter 200. Outer tube 204 is dimensioned for endoscopic insertion, in particular, outer tube 204 is passable through a typical trocar port, cannula, or the like. Knob housing 202 is dimensioned to not enter the trocar port, cannula, or the like. Knob housing 202 is configured and adapted to connect to connecting portion 104 of handle housing 102 of surgical device 100.

Adapter 200 includes a plurality of force/rotation transmitting/converting assemblies disposed therein. Each force/rotation transmitting/converting assembly is configured and adapted to transmit/convert a speed/force of rotation (e.g., increase or decrease) of first, second and third drive connectors 106, 108, 110 of surgical device 100 before transmission of such rotational speed/force to loading unit 300.

Adapter 200 further includes an attachment/detachment button 214 supported thereon. Specifically, button 214 is supported on drive coupling assembly 212 of adapter 200 and is biased to an un-actuated condition. Button 214 includes at least one lip or ledge 214a formed therewith that is configured to snap behind a corresponding lip or ledge 104b defined along recess 104a of connecting portion 104 of surgical device 100. In use, when adapter 200 is connected to surgical device 100, lip 214a of button 214 is disposed behind lip 104b of connecting portion 104 of surgical device 100 to secure and retain adapter 200 and surgical device 100 with one another. In order to permit disconnection of adapter 200 and surgical device 100 from one another, button 214 is depresses or actuated, against its bias condition, to disengage lip 214a of button 214 and lip 104b of connecting portion 104 of surgical device 100.

Adapter 200 includes an electrical assembly 216 supported on and in outer knob housing 202. Electrical assembly 216 includes a plurality of electrical contact pins 218, supported on a circuit board (not shown), for electrical connection to a corresponding electrical plug 112 disposed in connecting portion 104 of surgical device 100. Electrical assembly 216 serves to allow for calibration and communication of life-cycle information to the circuit board of surgical device 100 via electrical plug 112 that are electrically connected to the circuit board (not shown) of surgical device 100.

For a detailed description of the construction and operation of exemplary electromechanical, hand-held, powered surgical instruments 100, adapters 200, and end effectors 300, reference may be made to International Application No. PCT/US2008/077249, filed September 22, 2008 (Inter. Pub. No. WO 2009/039506), U.S. Patent Publication No. 2009/0314821, filed on August 31, 2009, U.S. Patent Publication No.2011/0121049, filed on November 20, 2009, U.S. Patent Publication No. 2013/0324978, filed on May 2, 2013, and U.S. Provisional Patent Application Serial No. 61/911,774, filed on December 4, 2013, the entire contents of each of which are incorporated herein by reference.

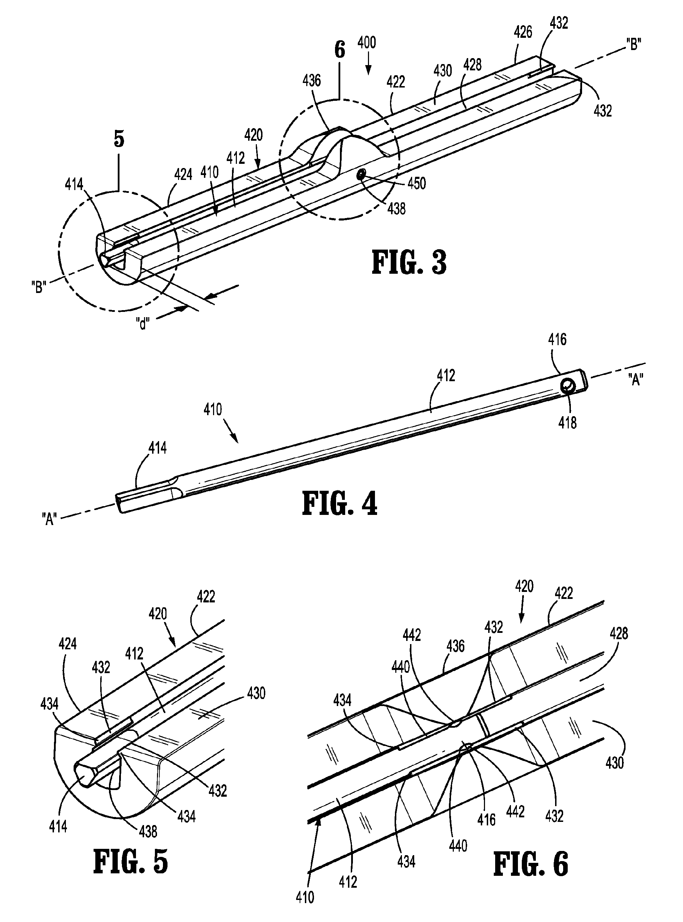

Turning now to FIGS. 3-7, an embodiment of a manual retraction tool 400 for use with an electromechanical surgical system, such as the one described above, includes a shaft 410 pivotally connected to a handle 420 by connector member 450. Shaft 410 includes an elongated shaft body 412 extending along a longitudinal axis "A." While shaft body 412 is shown as having a substantially cylindrical shape, it should be understood that shaft body 412 may be any shape within the purview of those skilled in the art. Shaft body 412 has a first end or tip 414 dimensioned and shaped to compliment the dimension and shape of a drive connector of an electrosurgical device, and a second end 416 including a through-hole 418 dimensioned to receive connector member 450. As shown, tip 414 has a tri-lobe shape which corresponds to tri-lobe tips 106a, 108a, 110a of drive connectors 106, 108, 110 of surgical device 100 (FIG. 2), but a person of ordinary skill will understand that the shape of tip 414 of shaft 410 may have a dimension and shape that compliments the dimension and shape of a drive connector of any surgical device, and/or any dimension and shape that will effect rotation of connector sleeves of the adapter with which it will be utilized. Thus, for purposes of discussion, tip 414 is illustrated throughout the embodiments of the present disclosure as having the same shape as tri-lobe tips 106a, 108a, 110a of drive connectors 106, 108, 110 of surgical device 100, described above, for ease of understanding.

Handle 420 includes an elongated handle body 422 including a first end 424 and a second end 426 extending along a longitudinal axis "B." A channel 428 is recessed within at least a portion of a surface 430 of handle body 422 and is dimensioned to receive at least a portion of shaft body 412. Channel 428 may be dimensioned and shaped to be complementary to the shape of shaft body 412. In certain embodiments, channel 428 may have the same diameter as shaft body 412 to frictionally engage shaft body 412, and in some embodiments, channel 428 may have a slightly larger diameter than shaft body 412 so that shaft body 412 may freely move in and out channel 428. As shown, channel 428 extends throughout the entire length of handle body 422 from first end 424 to second end 426, however, it should be understood that channel 428 may extend only partially through handle body 422. Handle body 422 has a cross-section, transverse to longitudinal axis "B," that defines a generally arcuate configuration, such as a c-shape, a u-shape, or a horseshoe-shape, although other shapes are envisioned to those skilled in the art.

Handle body 422 includes retaining features 432 at first and second ends 424, 426 extending at least partially into and/or over channel 428. Retaining features 432 may be ridges, ribs, protrusions, bumps, projections, lips, flanges, overhangs, etc. that retain shaft body 412 within channel 428 of handle 420. As specifically shown in FIG. 5, first end 424 includes retaining features in the form of ridges 432 extending from opposed lateral edges 434 of handle body 422 that project into channel 438 thereby narrowing a transverse dimension of channel 428 for releasably retaining shaft body 412 therein. In embodiments, handle body 420 may include a single retaining feature 432, such as single ridge on either lateral edge 434, and/or retaining feature(s) 432 on only one of first and second ends 424, 246.

Handle body 422 includes a central portion 436 defined between the first and second ends 424, 426. Central portion 436 includes through-holes 438 that are transversely spaced and longitudinally aligned on opposing sides of channel 428. Through-hole 418 of shaft body 412 is aligned with through-holes 438 of handle body 422 such that connector member 450 can be transversely inserted therethough to pivotally connect shaft 410 and handle 420. Connector member 450 may be a pivot pin, a rivet, a bolt, among other fasteners within the purview of those skilled in the art for pivotally or rotatably connecting shaft 410 and handle 420. In certain embodiments, connector member 450 may be a feature for overmold retention.

As specifically shown in FIG. 6, central portion 436 of handle body 422 includes retaining members 440 extending at least partially into and/or over channel 428. Retaining members 440 include ridges 441 (not shown) extending from opposed lateral edges 434 that project into channel 438, like retaining features 432 defined at first and second ends 424, 426 of handle body 422, described above. Unlike retaining features 432, however, retaining members 440 include grooves 442 aligned with through-holes 438 along an axis perpendicular to longitudinal axis "B" that, together are dimensioned to snugly receive and retain shaft body 412 at about a 90° angle with respect to handle body 422. Grooves 442 may form a complementary shape to shaft body 412 and be dimensioned to be the same size as the outer surface of shaft body 412. It is envisioned that retaining members 440 may be configured to retain shaft 410 at other angles with respect to handle 420.

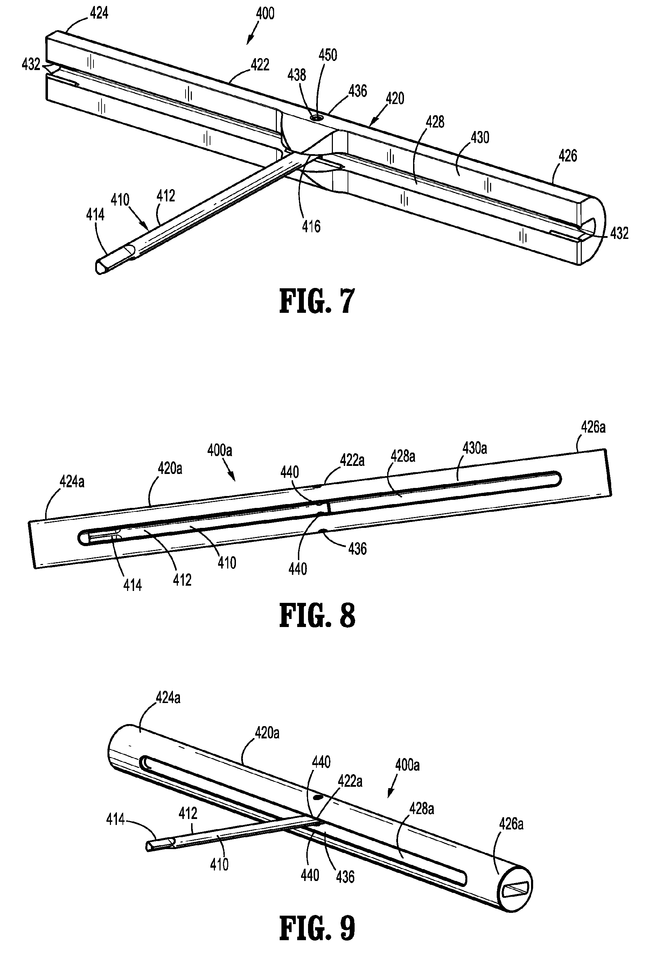

Shaft 410 may be disposed within channel 428 of handle 420 in a first position as shown in FIG. 3. In the first position, shaft body 412 is longitudinally aligned with handle body 422 such that longitudinal axis "A" is coincident with longitudinal axis "B." Tip 414 of shaft 410 extends out of first end 424 of handle 420 a distance "d" that corresponds to the distance tri-lobe tips 106a, 108a, 110a of drive connectors 106, 108, 110 extend beyond wall 103 of connecting portion 104 of surgical device 100 (FIG. 2). Shaft 410 may be pivoted, with respect to handle 420 about connector member 450, to a second position, as shown in FIG. 7. In the second position, shaft body 412 is substantially perpendicular to handle body 422 such that longitudinal axis "A" is orthogonal to longitudinal axis "B." It should be understood that the shaft may be rotated to a third position that mirrors the first position. In the third position, shaft body 412 is disposed within channel 428 and longitudinally aligned with handle body 422, with tip 414 of shaft 410 extending out of second end 426 of handle 420.

During use of electromechanical surgical system 1, if surgical device 100 malfunctions, a clinician can detach surgical device 100 from adapter 200 via attachment/detachment button 214, as described above. The clinician can then utilize manual retraction tool 400 in any of the three positions, described above, by pivoting shaft 410 about connector member 450 and gripping handle body 422 for manually rotating tip 414 of shaft 410 within recess 206a, 208a, 210a of connector sleeve 206, 208, 210 of adapter 200 to effect actuation of a select function of end effector 300 (e.g., complete a firing stroke, reverse a firing stroke, reverse a clamp, open and/or articulate a tool assembly, rotate the end effector etc.). The clinician, for example, may choose to utilize manual retraction tool 400 in the first or third position to imitate the attachment of surgical device 100 to adapter 200 such that the outermost end of first end 424 of handle body 422 is flush against adapter 200, or in the second position to either increase the magnitude of the moment of the applied force by increasing the moment arm, or to generate the same moment as that of the first and third positions by applying less force to rotate manual retraction tool 400, thereby making rotation easier.

FIGS. 8 and 9 illustrate an alternative embodiment of the manual retraction tool 400 of FIGS. 3-7, shown generally as 400a. Manual retraction tool 400a is substantially similar to manual retraction tool 400, and therefore will only be described with respect to the differences therebetween. In contrast to manual retraction tool 400, tip 414 of shaft 410 does not extend out of first end 424a or second end 426a of handle 420a when in the first position or third position, but rather, is retained within channel 428a. Accordingly, channel 428a need only extend along the length of shaft 410, and not necessarily through the entire length of handle 420a. As shown, channel 428a does not extend through first and second ends 424a, 426a of handle body 422a. In such embodiments, retaining features 432 may be omitted for retaining shaft 410 in the first and third positions. In use, a clinician may utilize manual retraction tool 400a in the second position (FIG. 9) by rotating shaft 410 out of channel 428a and into the second position, where shaft body 412 is retained at about a 90° angle with respect to handle body 422a via retaining member 440.

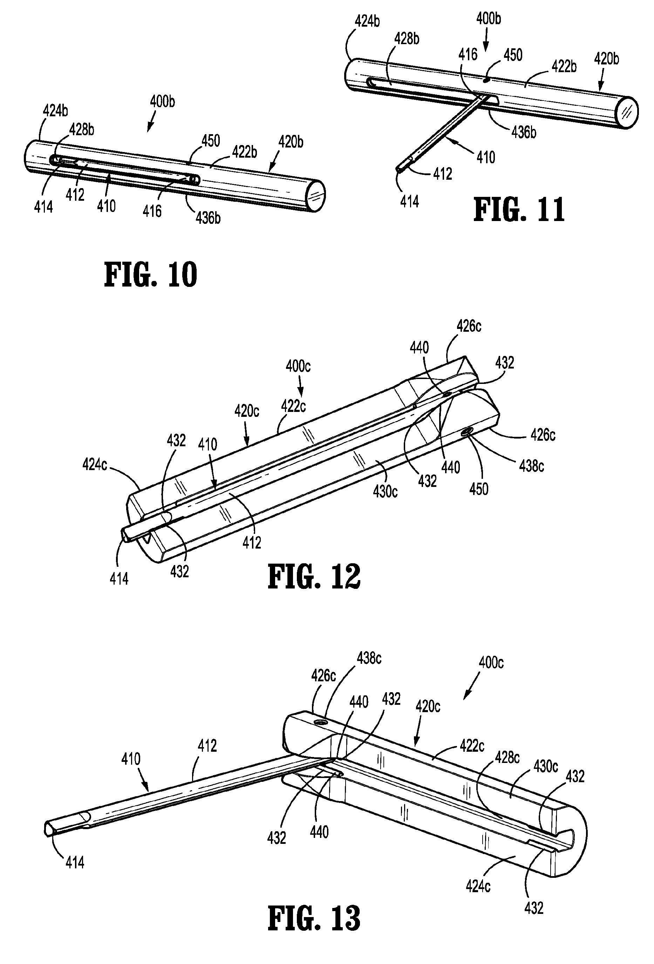

In yet an alternative embodiment of manual retraction tool 400a, shown in FIGS. 10 and 11, tip 414 of shaft 410 does not extend out of first end 424b of handle 420b when in the first position, but rather, is retained within channel 428b. Channel 428b extends only partially along the length of handle 420b and terminates at central portion 436b. It should be understood that channel 438b of handle 420b and/or shaft 410 need not necessarily terminate at central portion 436b but may terminate along any length of handle 420b. Thus, manual retraction tool 400b is rotatable about connector member 450 between a first position in which shaft body 412 is retained within channel 428b and longitudinally aligned with handle body 422b (FIG. 10), and a second position in which shaft body 412 extends at about a 90° angle with respect to handle body 422b for use by a clinician (FIG. 11).

Turning now to FIGS. 12-14, a manual retraction tool 400c in accordance with another embodiment includes shaft 410 and a handle 420c. Handle 420c includes an elongated handle body 422c including a first end 424c and a second end 426c. A channel 428c is recessed and within at least a portion of a surface 430c of handle body 422c, and is dimensioned to receive at least a portion of shaft body 412. As shown, channel 428c extends throughout the entire length of handle body 422c. Handle body 422c includes retaining features 432 at first end 424c and a retaining member 440 at second end 426c. Second end 426c also includes through-holes 438c that are transversely spaced and longitudinally aligned on opposing sides of channel 428c. Through-hole 418 of shaft body 412 (FIG. 4) is aligned with through-holes 438c of handle body 422c such that connector member 450 can be transversely inserted therethough to pivotally connect shaft 410 and handle 420c.

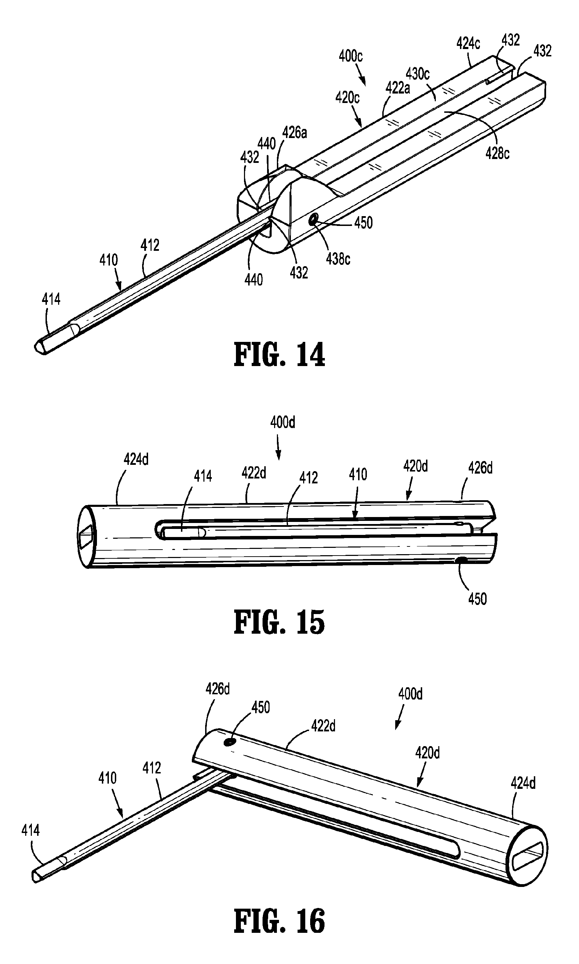

As shown in FIG. 12, in a first position, shaft body 412 is longitudinally aligned with handle body 422c and retained within channel 428c via retaining features 432. Tip 414 of shaft 410 extends out of first end 424c of handle body 422c in the same manner as that shown in FIG. 3. Shaft 410 may be pivoted, with respect to handle 420c, to a second position, as shown in FIG. 13. In the second position, shaft body 412 is substantially perpendicular to handle body 422c and retained in the second position via grooves 422 (not shown) in retaining members 440. As shown in FIG. 14, shaft 410 may be pivoted to a third position in which shaft body 412 is longitudinally aligned with handle body 422c, with a majority of shaft body 412 extending out of second end 426c of handle body 422c. Shaft 410 is retained in the third position via ridges 441 of retaining members 440. Accordingly, in use, a clinician may utilize manual retraction tool 400c in any of the three positions to vary the moment arm and/or applied forced required to rotate connector sleeve 206, 208, 210 of adapter 200 when tip 414 of shaft 410 is inserted into recess 206a, 208a, 210a of connector sleeve 206, 208, 210, as described above.

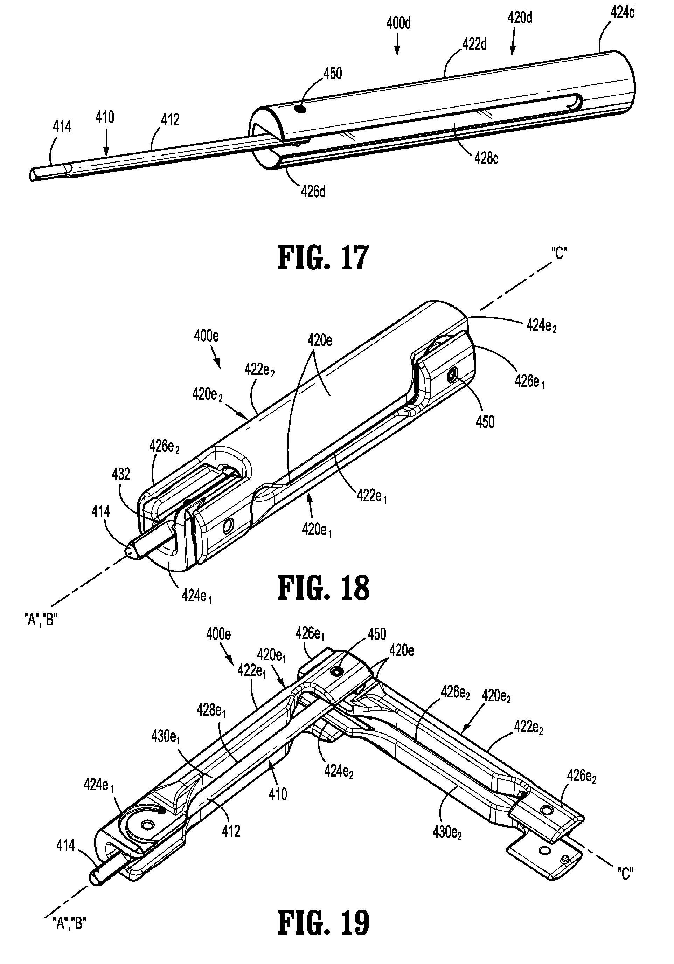

FIGS. 15-17 illustrate an alternative embodiment of manual retraction tool 400c of FIGS. 12-14, shown generally as 400d. Manual retraction tool 400d is substantially similar to manual retraction tool 400c, and therefore will only be described with respect to the differences therebetween. In contrast to manual retraction tool 400c, tip 414 of shaft 410 does not extend out of the first end 424d of handle body 422d of manual retraction tool 400d when in the first position, but rather is retained within channel 428d. Accordingly, channel 428d need only extend along the length of shaft 410, and not necessarily through the entire length of handle 420d. As shown, channel 428d does not extend through first ends 424d of handle body 422d. In such embodiments, retaining features 432 may be omitted from first end 424d for retaining shaft 410 in the first position (FIG. 15). In use, a clinician rotates shaft 410 out of channel 428d about connector member 450 and into either the second position (FIG. 16), where shaft body 412 is substantially perpendicular to handle body 422d, or to the third position (FIG. 17), where shaft body 412 is longitudinally aligned with handle body 422d.

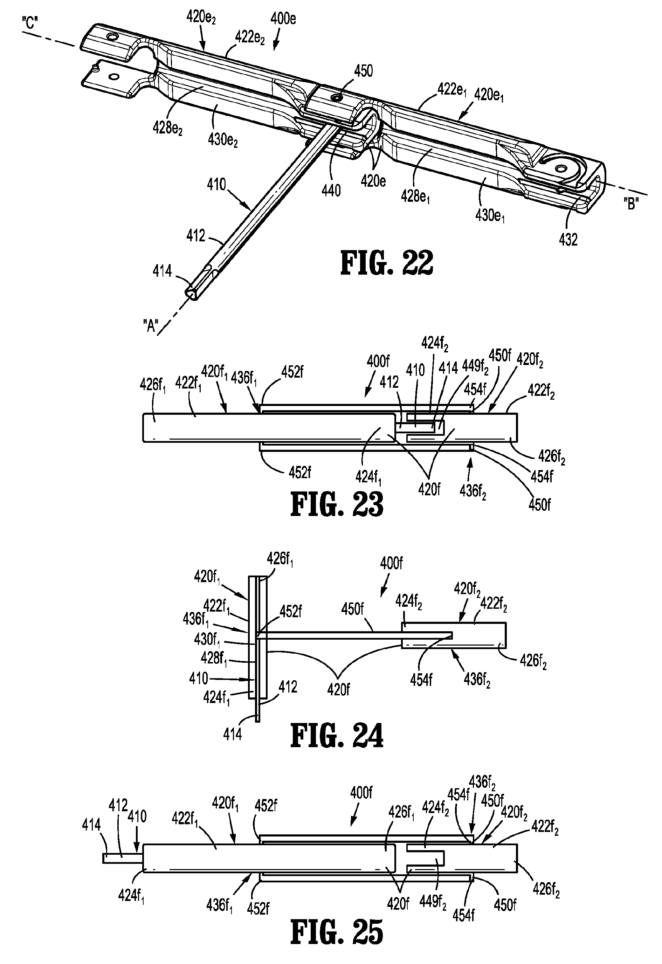

Turning now to FIGS. 18-22, a manual retraction tool 400e in accordance with another embodiment of the present disclosure includes shaft 410 connected to a two-piece handle assembly 420e by connector member 450. Handle assembly 420e includes a first handle 420e₁ and a second handle 420e₂ that each include, respectively, an elongated handle body 422e₁, 422e₂ including a first end 424e₁, 424e₂ and a second end 426e₁, 426e₂ extending along longitudinal axes "B" and "C." A channel 428e₁, 428e₂ is recessed within at least a portion of a surface 430e₁, 430e₂ of each of first and second handle bodies 422e₁, 422e₂ to accommodate shaft 410, as described above. First end 424e₁ of first handle body 422e₁ includes retaining features 432, and first end 424e₂ of second handle body 422e₂ includes retaining members 440 for retaining shaft 410 in a first position (FIGS.18-21) or a second position (FIG. 22), respectively.

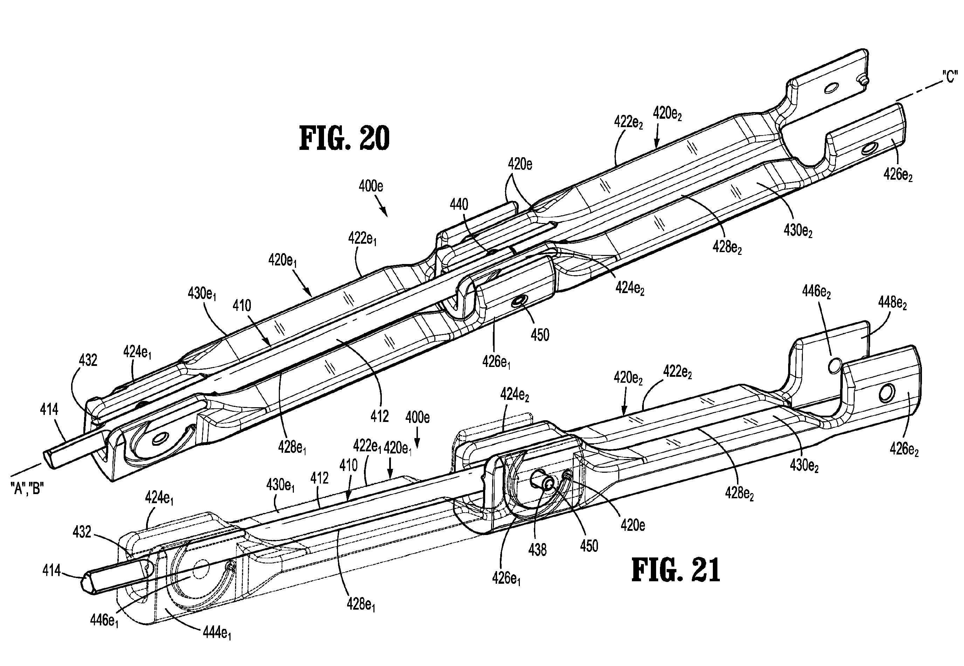

As specifically shown in FIG. 21, second end 426e₁ of first handle body 422e₁ is dimensioned to receive first end 424e₂ of second handle body 422e₂, with each of second end 426e₁ and first end 424e₂ including through-holes 438 that are transversely spaced and longitudinally aligned on opposing on opposing sides of channel 428e₁, 428e₂. Through-hole 418 of shaft body 412 (FIG. 4) is aligned with through-holes 438 such that connector member 450 can be transversely inserted therethrough such that shaft 410, first handle 420e₁, and second handle 420e₂ are all independently pivotable/rotatable with respect to each other.

An outer surface 444e₁ of first end 424e₁ of first handle body 422e₁ includes releasable engagement parts 446e₁ for engaging complementary engagement parts 446e₂ on an inner surface 448e₂ of second end 426e₂ of second handle body 422e₂ in a snap fit relation. It should be understood that any releasable mating structure may be utilized, such as friction fit, tongue and groove arrangements, etc.

In a first position, as shown in FIGS. 18-21, shaft 410 is disposed within channel 428e₁ of first handle 420e₁ and longitudinally aligned with first handle body 422e₁ such that longitudinal axis "A" of shaft 410 is coincident with longitudinal axis "B" of first handle 420e₁. In the first position, tip 414 of shaft 410 longitudinally extends out of and beyond first end 424e₁ of first handle body 422e₁ a distance that is the same distance tip 106a, 108a, 110a of drive connector 106, 108, 110 extend beyond wall 103 of connecting portion 104 of surgical device 100 (FIG. 2). First and second handles 420e₁, 420e₂ may be positioned in a closed position (FIG. 18) in which first and second handles 420e₁, 420e₂ are mated via engagement parts 446e₁, 446e₂, and longitudinal axis "B" of first handle body 422e₁ is coincident with longitudinal axis "C" of second handle body 422e₂. First and second handles 420e₁, 420e₂ may be positioned in a semi-open position (FIG. 19) in which second handle body 422e₂ is pivoted about connector member 450 to a position in which longitudinal axis "C" of second handle body 422e₂ is perpendicular to longitudinal axis "B" of first handle body 422e₁. First and second handles 420e₁, 420e₂ may be position in a fully open position (FIGS. 20 and 21), wherein longitudinal axes "B" and "C" of handle bodies 422e₁, 422e₂ are longitudinally aligned and extend along the same axis.

In a second position, as shown in FIG. 22, shaft 410 extends at about a 90° angle with respect to first and second handle bodies 422e₁, 422e₂, such that longitudinal axis "A" is orthogonal to longitudinal axes "B" and "C." Accordingly, manual retraction tool 400e includes two positions of shaft 410, and three positions of first and second handles 422e₁, 422e₂. Thus, a clinician may utilize manual retraction tool 400e in a desired position to generate the moment for rotating connector sleeves 206, 208, 210 of adapter 200.

FIGS. 23-25 illustrate a manual retraction tool 400f in accordance with yet another embodiment of the present disclosure. Manual retraction tool 400f includes shaft 410 and a two-piece handle assembly 420f connected by connection member 450f. Handle assembly 420f includes a movable first handle 420f₁ including an elongated first handle body 422f₁ having a first end 424f₁ and a second end 426f₁. A channel 428f₁ is recessed within at least a portion of a surface 430f₁ of first handle body 422f₁ and is dimensioned to receive at least a portion of shaft body 412. As shown, shaft 410 is fixed within first handle body 422f₁ such that tip 414 of shaft 410 longitudinally extends out of first end 424f₁ of first handle body 422f₁ a distance "d" that is the same distance tip 106a, 108a, 110a of drive connector 106, 108, 110 extends distally beyond wall 103 of connecting portion 104 of surgical device 100 (FIG. 2). Alternatively, shaft 410 may be pivotally disposed within first handle body 422f₁ in a manner similar to that of FIGS. 3-7.

Handle assembly 420f includes a non-movable or fixed second handle 420f₂ including an elongated second handle body 422f₂ having a first end 424f₂ and a second end 426f₂. First end 424f₂ of second handle body 422f₂ includes a u-shaped notch 449f₂ at an outermost end thereof that is dimensioned to receive tip 414 of shaft 410. It should be understood that other shapes and dimensions of notch 449f₂ are envisioned. Second end 426f₂ is dimensioned for gripping by a clinician.

First and second handles 420f₁, 420f₂ are connected via elongated rods 450f. Rods 450f are attached to first and second handle bodies 422f₁, 422f₂ about a central portion 436f₁, 436f₂ of first and second handles 420f₁, 420f₂. It is envisioned, however, that rods 450f may be attached about any portion along the length of first and second handles 420f₁, 420f₂. Rods 450f include a first end 452f pivotally attached to first handle body 422f₁ and a second end 454f non-pivotally secured to second handle body 422f₂.

As shown in FIG. 23, in a retracted position, first end 424f₁ of first handle 420f₁ is proximate to first end 424f₂ of second handle 420f2 such that tip 414 of shaft 410 is disposed within notch 449f₂ of second handle body 422f₂. First handle 422f₁ may be pivoted about first end 452f of rods 450f by about 90° to a semi-extended position in which first handle body 422f₁ and shaft body 412 are substantially perpendicular and longitudinally spaced from second handle body 422f₂, as shown in FIG. 24. First handle 422f₁ may be pivoted about the first end 452f of rods 450f by another 90 ° from the semi-extended position, or 180° from the retracted position, to a fully extended position in which the first and second handle bodies 422f₁, 422f₂ are longitudinally aligned and tip 414 of shaft 410 extends out and away from second handle 422f₂, as shown in FIG. 25. In use, a clinician may utilize manual retraction tool 400f in the semi-extended position, such as in situations where space/access is restricted, or in the fully extended position.

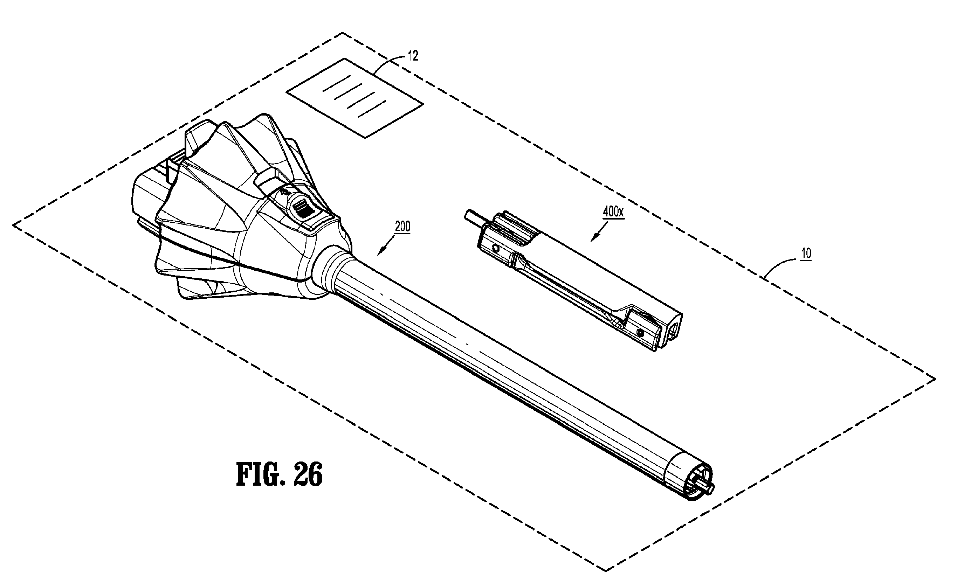

The present disclosure contemplates, as seen in FIG. 26, kits including any one or more of the aforedescribed manual retraction tools 400ₓ and an adapter 200, with or without an end effector and/or a surgical device. A kit may also include packaging 10 for containing the manual retraction 400ₓ and adapter 200, such as trays, blister packs, pouches, and/or boxes, among other containers within the purview of those skilled in the art. In embodiments, the outer diameter of a handle of a manual retraction tool may match the outer diameter of an outer tube of an adapter for tray compatibility. A kit may include instructions for use 12 setting forth a method of use, such as the ones described above. The instructions for use 12 may be provided on and/or within the packaging. The manual retraction 400ₓ and adapter 200 may be provided in a sterile condition.

It will be understood that various modifications may be made to the embodiments disclosed herein. For example, the shape of a handle may be modified, e.g., to include grips, for ease of handling by a clinician. Therefore, the above description should not be construed as limiting, but merely as exemplifications of preferred embodiments. Those skilled in the art will envision other modifications within the scope and spirit of the claims appended thereto.

The invention may be described by reference to the following numbered paragraphs:-
1. A manual retraction tool for use with an electromechanical surgical assembly including a surgical device including at least one drive connector disposed within a connecting portion, an adapter including at least one connector sleeve defining a recess therein configured to receive the drive connector of the surgical device, and an end effector attached to the adapter, the manual retraction tool comprising:
   a handle including an elongated handle body having a first end and a second end, and including a channel recessed within at least a portion of the handle body; and
   a shaft including an elongated shaft body having a tip dimensioned to be inserted into the recess of a connector sleeve of an adapter and to effect rotation of a connector sleeve upon rotation of the shaft, and a second end pivotally connected to the handle body, the shaft body being movable between a first position wherein the shaft is at least partially disposed within the channel of the handle body and longitudinally aligned with the handle body, and a second position wherein the shaft body is perpendicular to the handle body.
2. The manual retraction tool according to paragraph 1, wherein the handle body includes through-holes transversely spaced and longitudinally aligned on opposing sides of the channel, and the second end of the shaft body includes a through-hole that is aligned with the through-holes of the handle, and a connector member extends transversely through the through-hole of the shaft body and the through-holes of the handle body to pivotally connect the shaft to the handle.
3. The manual retraction tool according to paragraph 2, wherein the through-holes of the handle body are disposed within a central portion of the handle body.
4. The manual retraction tool according to paragraph 2, wherein the through-holes of the handle body are disposed within the second end of the handle body.
5. The manual retraction tool according to paragraph 1, wherein the second end of the shaft body is pivotally connected to a central portion of the handle body.
6. The manual retraction tool according to paragraph 5, wherein the channel extends along an entire length of the handle body, and wherein the shaft body is movable to a third position that mirrors the first position.
7. The manual retraction tool according to paragraph 1, wherein the second end of the shaft is pivotally connected the second end of the handle body.
8. The manual retraction tool according to paragraph 7, wherein the shaft is movable to a third position in which a majority of the shaft extends out of the second end of the shaft body.
9. The manual retraction tool according to paragraph 1, wherein the tip of the shaft extends out of the first end of the handle body when in the first position.
10. The manual retraction tool according to paragraph 9, wherein the tip of the shaft extends a distance that is a same distance that the drive connector extends from the connecting portion of the surgical device.
11. The manual retraction tool according to paragraph 1, wherein the first end of the handle body includes retaining features for retaining the shaft in the first position.
12. The manual retraction tool according to paragraph 1, wherein the tip of the shaft is retained within the channel of the handle body when in the first position.
13. The manual retraction tool according to paragraph 1, wherein the handle body includes retaining members for retaining the shaft in the second position.
14. The manual retraction tool according to paragraph 1, further comprising a handle assembly wherein the handle is a first handle of the handle assembly, the handle assembly including a second handle including an elongated second handle body having a first end and a second end, the second handle being pivotally connected to the first handle such that the shaft, the first handle, and the second handle are all independently pivotable with respect to each other.
15. The manual retraction tool according to paragraph 14, wherein the handle assembly is pivotable to a closed position in which the first and second handles mate with each other and longitudinal axes of the first and second handle bodies are coincident with each other.
16. The manual retraction tool according to paragraph 15, wherein the first and second handle bodies include complementary engagement parts for releasably retaining the first and second handles in the closed position.
17. The manual retraction tool according to paragraph 14, wherein the handle assembly is pivotable to a semi-open position in which the first and second handle bodies are orthogonal to each other.
18. The manual retraction tool according to paragraph 14, wherein the handle assembly is pivotable to a fully open position in which the first and second handle bodies longitudinally extend along an axis.
19. A method of using the manual retraction tool of paragraph 1, comprising the steps of:
   inserting the tip of the shaft into a connector sleeve of an adapter; and
   rotating the manual retraction tool to effect rotation of a connector sleeve.
20. The method of paragraph 19, further comprising the step of disconnecting the surgical device from the adapter prior to inserting the tip of the shaft into the connector sleeve.

## Claims

1. A manual retraction tool for use with an electromechanical surgical assembly including a surgical device including at least one drive connector disposed within a connecting portion, an adapter including at least one connector sleeve defining a recess therein configured to receive the drive connector of the surgical device, and an end effector attached to the adapter, the manual retraction tool comprising:
a handle including an elongated handle body having a first end and a second end, and including a channel recessed within at least a portion of the handle body; and
a shaft including an elongated shaft body having a tip dimensioned to be inserted into the recess of a connector sleeve of an adapter and to effect rotation of a connector sleeve upon rotation of the shaft, and a second end pivotally connected to the handle body, the shaft body being movable between a first position wherein the shaft is at least partially disposed within the channel of the handle body and longitudinally aligned with the handle body, and a second position wherein the shaft body is perpendicular to the handle body.

2. The manual retraction tool according to claim 1, wherein the handle body includes through-holes transversely spaced and longitudinally aligned on opposing sides of the channel, and the second end of the shaft body includes a through-hole that is aligned with the through-holes of the handle, and a connector member extends transversely through the through-hole of the shaft body and the through-holes of the handle body to pivotally connect the shaft to the handle.

3. The manual retraction tool according to claim 2, wherein the through-holes of the handle body are disposed within a central portion of the handle body; and/or wherein the through-holes of the handle body are disposed within the second end of the handle body.

4. The manual retraction tool according to any preceding claim, wherein the second end of the shaft body is pivotally connected to a central portion of the handle body; preferably wherein the channel extends along an entire length of the handle body, and wherein the shaft body is movable to a third position that mirrors the first position.

5. The manual retraction tool according to any preceding claim, wherein the second end of the shaft is pivotally connected the second end of the handle body; preferably wherein the shaft is movable to a third position in which a majority of the shaft extends out of the second end of the shaft body.

6. The manual retraction tool according to any preceding claim, wherein the tip of the shaft extends out of the first end of the handle body when in the first position.

7. The manual retraction tool according to claim 6, wherein the tip of the shaft extends a distance that is a same distance that the drive connector extends from the connecting portion of the surgical device.

8. The manual retraction tool according to any preceding claim, wherein the first end of the handle body includes retaining features for retaining the shaft in the first position.

9. The manual retraction tool according to any preceding claim, wherein the tip of the shaft is retained within the channel of the handle body when in the first position; and/or wherein the handle body includes retaining members for retaining the shaft in the second position.

10. The manual retraction tool according to any preceding claim, further comprising a handle assembly wherein the handle is a first handle of the handle assembly, the handle assembly including a second handle including an elongated second handle body having a first end and a second end, the second handle being pivotally connected to the first handle such that the shaft, the first handle, and the second handle are all independently pivotable with respect to each other.

11. The manual retraction tool according to claim 10, wherein the handle assembly is pivotable to a closed position in which the first and second handles mate with each other and longitudinal axes of the first and second handle bodies are coincident with each other.

12. The manual retraction tool according to claim 10 or claim 11, wherein the first and second handle bodies include complementary engagement parts for releasably retaining the first and second handles in the closed position.

13. The manual retraction tool according to claim 10, wherein the handle assembly is pivotable to a semi-open position in which the first and second handle bodies are orthogonal to each other.

14. The manual retraction tool according to claim 10, wherein the handle assembly is pivotable to a fully open position in which the first and second handle bodies longitudinally extend along an axis.

15. A method of using the manual retraction tool of any preceding claim, comprising the steps of:
inserting the tip of the shaft into a connector sleeve of an adapter; and
rotating the manual retraction tool to effect rotation of a connector sleeve; preferably further comprising the step of disconnecting the surgical device from the adapter prior to inserting the tip of the shaft into the connector sleeve.
